# EUROPEAN PATENT APPLICATION

(11) **EP 3 090 735 A1**
(43) Date of publication of application: **09.11.2016**
(21) Application number: 14877125.6
(22) Date of filing: 30.07.2014
(51) Int. Cl.: A61K 9/48, A61K 47/38, A61K 47/10, A61K 47/30

(54) **AQUEOUS COMPOSITION FOR HARD CAPSULE, AND HARD CAPSULE PRODUCED USING SAME**

(30) Priority: 31.12.2013 KR 20130168261
(71) Applicant: LOTTE Fine Chemical Co., Ltd., Ulsan, 44714 (KR)
(72) Inventor: SON, Jin Ryul, Incheon 406-736 (KR); CHUN, Jeong Hee, Incheon 401-731 (KR)
(74) Representative: Kador & Partner
(86) International application number: PCT/KR2014/006992
(87) International publication number: WO 2015/102196

(57) **Abstract**

The present invention relates to an aqueous composition for a hard capsule, the composition having an improved degree of gelation, and to a hard capsule produced using the aqueous composition. The present invention enables the production of an aqueous composition for a hard capsule, the composition having an excellent degree of gelation due to comprising a gelation agent and a gelation auxiliary agent along with water-soluble cellulose ether, alcohol and water, and as a result a high quality hard capsule having improved hard capsule moldability can be produced. Furthermore, the alcohol improves the solubility of the water-soluble cellulose ether, and thus a stable gel power can be maintained by only a small amount of gelation agent, thereby resolving the problem of the gelation agent causing a drop in solubility time.

## Description

### [Technical Field]

The present invention relates to an aqueous composition for a hard capsule having an improved degree of gelation, and a hard capsule produced using the same.

### [Background Art]

Capsules used for medical supplies and health functional foods are generally produced using gelatin or cellulose ether as a base.

Gelatin capsules have advantages such as high industrial productivity and price competitiveness, but also have a drawback in that the plasticity may be lost and the impact resistance may be significantly deteriorated when a moisture concentration is less than or equal to 10% by weight. Also, since the current use of gelatin has been limited due to problems such as bovine spongiform encephalopathy, capsules produced using a plant material such as cellulose ether rather than the gelatin have come into the spotlight.

Generally, methods for producing a hard capsule may be mainly divided into two methods such as a Hot pin process and a Cold pin process, depending on gel characteristics.

First of all, the Hot pin process uses a property of a cellulose ether solution being gelled when the cellulose ether solution is heated to a high temperature, and thus is a method of dipping a high-temperature mold pin into a cellulose ether solution maintained at a temperature higher than room temperature and thermally gelling the solution coated onto the pin through heat of the pin to produce a hard capsule.

As such a conventional method, Patent Document 1 (Registered US Patent No. 2,526,683) discloses a method of producing a medicinal methyl cellulose capsule using a dip coating method. Such a method includes dipping a mold pin, which has been preheated at 40 to 85°C, into a methyl cellulose composition maintained at a temperature lower than a temperature at which gelation starts to occur, recovering the pin, placing the pin in an oven at a temperature higher than a gelation point, and drying the resulting film. When the high-temperature pin is dipped into the composition, the composition is gelled on a surface of the pin. When the pin is recovered, a film made from a gelled liquid and having a predetermined thickness is formed on the pin. Subsequently, the pin is generally rotated at an angle of 180° to a vertical posture, and typically placed in an oven to be dried. Then, the dried capsule is peeled off, cut into pieces of a certain size, and fitted with a cap and a body portion. However, methyl cellulose is insoluble in water having a temperature of less than 37°C.

Next, the Cold pin process includes heating a solution prepared from gelatin which is gelled at room temperature, or a hydroxypropyl methyl cellulose (HPMC) solution including a compound, such as carrageenan, which is gelled at room temperature (a gelation agent), followed by aging the solution at a constant high temperature, dipping a cold mold pin into the solution to coat the mold pin with a predetermined amount of the solution, removing the mold pin from the solution, immediately exposing the solution coated on the mold pin to cold blown air having a temperature of approximately 20°C to form a gel, and drying the gel to produce a capsule. The gelation agent used in the Cold pin process has been generally used to produce capsules with metal ions such as potassium, calcium, and sodium to improve a gel-forming ability of the gelation agent. However, when a capsule to which a gelation agent such as carrageenan is added is orally administered, the gelation agent has a problem in that it re-reacts with metal salts present in gastric or intestinal juices to enhance a binding strength between capsule components, which makes it impossible to disintegrate the capsule. That is, an initial solubility time of the hard capsule is shown to be low due to an ionic characteristic of the gelation agent, and the hard capsule may exhibit other dissolution characteristics, depending on each medium.

To solve these problems, as a plan to reduce concentrations of the gelation agent and a gelation auxiliary agent, Patent Document 2 (Registered US Patent No. 5,756,123) discloses a capsule film composition which includes 18 to 28 parts by weight of HPMC, 0.01 to 0.1 parts by weight of carrageenan as the gelation agent, and 0.05 to 0.6 parts by weight of potassium or calcium ions as the gelation auxiliary agent. However, even though the solubility time is improved by such a method, the method has a drawback in that moldability of the hard capsule may be deteriorated due to a significant decrease in the degree of gelation of the hard capsule.

### [Prior-Art Documents]

### [Patent Documents]

(Patent Document 1) US2526683 B
(Patent Document 2) US5756123 B

### [Disclosure]

### [Technical Problem]

Therefore, the present invention is designed to solve the problems of the prior art, and therefore it is an object of the present invention to provide an aqueous composition for a hard capsule having a high degree of gelation to improve moldability of a hard capsule.

It is another object of the present invention to provide a hard capsule produced using the aqueous composition for a hard capsule.

### [Technical Solution]

To solve the above problems, according to an aspect of the present invention, there is provided an aqueous composition for a hard capsule which includes a first ingredient composed of a water-soluble cellulose ether, an alcohol and water; and a second ingredient including a gelation agent and a gelation auxiliary agent, wherein a coating film, which is formed while the aqueous composition for a hard capsule flows down when a mold pin (6Ø, 14.5 cm) kept at room temperature is dipped into the aqueous composition for a hard capsule to a depth of 2.5 cm, removed therefrom and flipped over, has a total length of 6.0 to 8.0 cm.

A concentration of the gelation agent may be in a range of 0.2 to 0.5 parts by weight, both exclusive, based on 100 parts by weight of the first ingredient, and a concentration of the gelation auxiliary agent may be in a range of 0.3 to 0.6 parts by weight, both exclusive, based on 100 parts by weight of the first ingredient. At least one water-soluble gum selected from the group consisting of carrageenan, gellan gum, xanthan gum, and pectin may be used as the gelation agent, and at least one selected from the group consisting of potassium chloride, potassium acetate, and calcium chloride may be used as the gelation auxiliary agent.

Preferably, a concentration of the water-soluble cellulose ether may be in a range of 10 to 25% by weight, based on 100% by weight of the first ingredient, and a concentration of the alcohol may be in a range of 5 to 30% by weight, based on 100% by weight of the first ingredient. At least one selected from the group consisting of hydroxypropyl methyl cellulose (HPMC), hydroxyethyl methyl cellulose (HEMC), and methyl cellulose (MC) may be used as the water-soluble cellulose ether, and at least one selected from the group consisting of ethanol, methanol, isopropanol, and butanol may be used as the alcohol.

According to another aspect of the present invention, there is provided a hard capsule produced using the aqueous composition for a hard capsule.

### [Advantageous Effects]

According to the present invention, an aqueous composition for a hard capsule which includes a gelation agent and a gelation auxiliary agent together with a water-soluble cellulose ether, an alcohol and water and thus has an excellent degree of gelation can be provided. As a result, a high-quality hard capsule having an improved hard capsule moldability may be provided.

Also, the alcohol improves the solubility of the water-soluble cellulose ether, and thus a stable gel power can be maintained only by a small amount of gelation agent, thereby solving the problem of the gelation agent causing a drop in a solubility time.

### [Best Mode]

The present invention is directed to an aqueous composition for a hard capsule which includes a first ingredient composed of a water-soluble cellulose ether, an alcohol and water; and a second ingredient including a gelation agent and a gelation auxiliary agent. Here, a coating film, which is formed while the aqueous composition for a hard capsule flows down when a mold pin (6Ø, 14.5 cm) kept at room temperature is dipped into the aqueous composition for a hard capsule to a depth of 2.5 cm, removed therefrom and flipped over, has a total length of 6.0 to 8.0 cm.

A degree of gelation of the aqueous composition for a hard capsule according to one exemplary embodiment of the present invention may be evaluated as the total length of the coating film. Here, the coating film having a shorter total length means that the coating film has a greater degree of gelation. When the total length of the coating film is out of this range, the aqueous composition may not be produced into capsules or quality may be deteriorated due to degraded moldability. The degree of gelation may be measured using various methods known in the art, for example, may be measured using a method of measuring a time (t = t) at which a flow of a solution coated onto a pin is stopped when the pin is dipped into a composition for a capsule for a predetermined period of time, removed therefrom and kept at room temperature (t = 0). In this case, however, it should be understood that the degree of gelation falls within the scope of the present invention when the degree of gelation is included in this range as measured by the method disclosed in the present invention.

A concentration of the gelation agent may preferably be in a range of 0.2 to 0.5 parts by weight, both exclusive, based on 100 parts by weight of the first ingredient. When the concentration of the gelation agent is within this range, a viscosity of the aqueous composition for a hard capsule may increase to a proper level, resulting in increased elongation at break and decreased brittleness of a hard capsule formed using the aqueous composition for a hard capsule. A water-soluble gum may be used as the gelation agent. Preferably, at least one selected from the group consisting of carrageenan, gellan gum, xanthan gum, and pectin may be used.

A concentration of the gelation auxiliary agent may preferably be in a range of 0.3 to 0.6 parts by weight, both exclusive, based on 100 parts by weight of the first ingredient. When the concentration of the gelation auxiliary agent is within this range, a gelling ability of the gelation agent may be improved, which makes it possible to obtain an aqueous composition for a hard capsule having excellent capsule moldability. At least one selected from the group consisting of potassium chloride, potassium acetate, and calcium chloride may be used as the gelation auxiliary agent.

The water-soluble cellulose ether is a main component of the aqueous composition for a hard capsule. Such a water-soluble cellulose ether is derived from cellulose that is a plant material, and thus has an advantage in that it is harmless to humans. In this specification, the term "cellulose ether" refers to a cellulose derivative obtained by etherifying a hydroxyl group of cellulose using an etherifying agent.

A concentration of the water-soluble cellulose ether may be in a range of 10 to 25% by weight, based on 100% by weight of the first ingredient. When the concentration of the water-soluble cellulose ether is in this range, bubbles may be easily removed since the aqueous composition may have a proper viscosity, and thus a capsule having a proper thickness may be obtained. At least one selected from the group consisting of hydroxypropyl methyl cellulose (HPMC), hydroxyethyl methyl cellulose (HEMC), and methyl cellulose (MC) may be used as the water-soluble cellulose ether.

The alcohol serves to aid in liquefying (that is, dissolving) the water-soluble cellulose ether in the aqueous composition for a hard capsule. Specifically, when the water-soluble cellulose ether is added to water having a low temperature (20 to 30°C), only a portion of the water-soluble cellulose ether coming in direct contact with the water is dissolved, but the other portion which does not come in direct contact with the water aggregates to form clusters. On the other hand, when the water-soluble cellulose ether is added to water having a high temperature (40 to 70°C), even a portion of the water-soluble cellulose ether coming in direct contact with the water tends not to be easily dissolved. However, the alcohol is mixed with water to form an aqueous alcohol solution, and the water-soluble cellulose ether is easily dissolved in an aqueous alcohol solution having a low temperature (20 to 30°C) as well as an aqueous alcohol solution having a high temperature (40 to 70°C).

A concentration of the alcohol may be in a range of 5 to 30% by weight, based on 100% by weight of the first ingredient. When the concentration of the alcohol is in this range, solubility of the cellulose ether may be enhanced, and an evaporation rate of the alcohol during production of the capsule may become appropriate, and thus a smooth capsule film having no wrinkles may be obtained. At least one selected from the group consisting of ethanol, methanol, isopropanol, and butanol may be used as the alcohol.

The second ingredient may further include a plasticizer at 0.05 to 5.0 parts by weight, both exclusive, based on 100 parts by weight of the first ingredient. When the concentration of the plasticizer is in this range, a hard capsule having a high elongation at break may be obtained. At least one selected from the group consisting of glycerol, sorbitol, propylene glycol, and polyethylene glycol may be used as the plasticizer.

Also, in the preparation of the aqueous composition, an emulsifying agent for improving moldability of the capsule may be additionally added to water to prepare the aqueous composition. Sodium lauryl sulfate (SLS), a sugar ester (SE) or a mixture thereof may be used as the emulsifying agent. In particular, SLS may significantly improve a capsule forming ability. A concentration of the emulsifying agent may be in a range of 0.01 to 1.0 parts by weight, and preferably, 0.05 to 0.5 parts by weight, both exclusive, based on 100 parts by weight of the first ingredient. When the concentration of the emulsifying agent is in this range, a decrease in drying property of the aqueous composition coated onto a mold pin may be realized, and thus a capsule having excellent moldability and good quality and exhibiting high stability with which the occurrence of gastroenteric troubles upon drug taking is suppressed may be obtained.

Hereinafter, one example of the method for preparing an aqueous composition for a hard capsule will be described in detail.

The method for preparing an aqueous composition according to one exemplary embodiment of the present invention may include mixing water and an alcohol to prepare an aqueous alcohol solution (S1), heating the aqueous alcohol solution (S2), adding a water-soluble cellulose ether to the heated aqueous alcohol solution to prepare a cellulose ether solution (S3), aging the cellulose ether solution (S4), and adding a gelation agent and a gelation auxiliary agent to the resulting solution (S5).

In the step S2, the heating of the aqueous alcohol solution may be performed at a temperature ranging from room temperature (20 to 30°C) to a temperature of 40 to 70°C. This step S2 is done to readily disperse the water-soluble cellulose ether in the aqueous alcohol solution so that the water-soluble cellulose ether is readily dissolved in the aqueous alcohol solution without aggregation in the step S3. When the heating temperature is in this range, an aqueous composition for a hard capsule having high capsule moldability and exhibiting a minimal increase in energy cost caused by inevitable heating may be obtained without solidifying a gelation agent and a gelation auxiliary agent to be described below.

The step S3 may be performed by slowly adding the water-soluble cellulose ether into the heated aqueous alcohol solution while stirring (for example, at 300 rpm).

The aging of the cellulose ether solution (S4) may be performed at a temperature of 40 to 70°C for 2 to 12 hours. When the water-soluble cellulose ether is completely dissolved by the aging, the aqueous composition has the following advantages: first, has a shorter production time; second, exhibits high homogeneity and uniform viscosity, and has no the layor separation of the solution even when stored for a long period of time; third, has a constant viscosity maintained for all production units; fourth, has high capsule moldability since insoluble products (for example, cellulose ether) suppressing the functions of a gelation agent and an optional gelation auxiliary agent do not exist; fifth, has high miscibility between cellulose ether and the gelation agent (and the optional gelation auxiliary agent), resulting in a decrease in the amount of the added gelation agent and gelation auxiliary agent; and, sixth, has high filtration efficiency in a subsequent filtration process for removing foreign matter from the aqueous composition for a hard capsule. In the step S5, the plasticizer may be additionally added to the resulting solution in addition to the gelation agent and the gelation auxiliary agent.

At least one of the steps S1 to S5 may be performed while stirring.

After the step S5, the method may further include removing bubbles from the aqueous composition for a hard capsule. The functions, types and concentrations of the alcohol, the water-soluble cellulose ether, the gelation agent, the gelation auxiliary agent and the plasticizer are as described above, and thus detailed description thereof are omitted.

However, the method for preparing an aqueous composition for hard capsule according to one exemplary embodiment of the present invention is not limited thereto, and may be widely modified by those skilled in the related art. For example, the cellulose ether solution prepared through the steps S1 to S3 may also be prepared through the following steps M1 to M3 or step N1.

That is, the cellulose ether solution may be prepared by mixing water and an alcohol to prepare an aqueous alcohol solution (M1), adding cellulose ether to the aqueous alcohol solution to prepare a cellulose ether solution (M2), and heating the cellulose ether solution to 40 to 70°C (M3).

Also, the cellulose ether solution may be prepared by mixing all of water, a water-soluble cellulose ether and an alcohol, each of which are heated to 40 to 70°C, to prepare a cellulose ether solution (N1).

According to the present invention, a hard capsule produced using the aqueous composition for a hard capsule is provided. For example, the hard capsule may be produced by dipping a mold pin kept at room temperature (20 to 30°C) into the aqueous composition for a hard capsule heated to a high temperature (40 to 70°C), removing the mold pin from the aqueous composition, and then drying the aqueous composition on the mold pin (this process is referred to as a 'cold pin process').

Hereinafter, the present invention will be described in further detail with reference to examples thereof. However, it should be understood that the description proposed herein is not intended to limit the scope of the present invention.

### Example 1

15 parts by weight of ethanol and 65 parts by weight of purified water were mixed to prepare an aqueous ethanol solution. Thereafter, the aqueous ethanol solution was heated to 60°C, and 20 parts by weight of hydroxypropyl methyl cellulose (HPMC) (Samsung Fine Chemicals Co., Ltd., AW4) was then added to the aqueous ethanol solution, dissolved therein, and aged for 4 hours to prepare a cellulose ether solution. Then, 0.5 parts by weight (exclusive) of K-carrageenan as a gelation agent, and 0.5 parts by weight (exclusive) of potassium chloride as a gelation auxiliary agent were added, based on 100 parts by weight of the cellulose ether solution, to prepare an aqueous composition for a hard capsule.

### Examples 2 to 5 and Comparative Examples 1 to 4

Hard capsules were produced in the same manner as in Example 1, except that the concentrations of the K-carrageenan and the potassium chloride were adjusted as listed in the following Table 1.

### Comparative Example 5

80 parts by weight of purified water was heated to 80°C, and 20 parts by weight of HPMC (Samsung Fine Chemicals Co., Ltd., AW4) was dispersed, and then cooled to 50°C. Thereafter, the resulting dispersion was warmed to 60°C to prepare a solution, and the solution was kept at 60°C for 24 hours to remove bubbles, thereby preparing a cellulose ether solution. Subsequently, 0.3 parts by weight (exclusive) of K-carrageenan as the gelation agent, and 0.5 parts by weight (exclusive) of potassium chloride as the gelation auxiliary agent were added, based on 100 parts by weight of the cellulose ether solution, to prepare an aqueous composition for a hard capsule.

### Experimental Example 1: Evaluation of degree of gelation

The total lengths of coating films, which were formed while the respective aqueous compositions prepared in Examples 1 to 5 and Comparative Examples 1 to 5 flowed down when mold pins (6Ø, 14.5 cm) for testing a degree of gelation at room temperature were dipped into each of the aqueous compositions to a depth of 2.5 cm, removed therefrom and flipped over, are listed in the following Table 1. The degree of gelation was evaluated as the total length of the coating film. Here, the coating film having a shorter total length means that the coating film has a greater degree of gelation.

**[Table 1]**

| | Concentration (% by weight) | | Total length (cm) of coating film |
|---|---|---|---|
| | K-carrageenan | Potassium chloride | |
| Example 1 | 0.5 | 0.5 | 6.5 |
| Example 2 | 0.4 | 0.5 | 6.5 |
| Example 3 | 0.3 | 0.5 | 7.0 |
| Example 4 | 0.4 | 0.4 | 7.5 |
| Example 5 | 0.5 | 0.4 | 6.5 |
| Comparative Example 1 | 1.5 | 0.5 | 3.5 |
| Comparative Example 2 | 0.5 | 0.2 | 9.0 |
| Comparative Example 3 | 2.0 | 0.0 | 10.0 |
| Comparative Example 4 | 3.0 | 1.0 | 2.5 |
| Comparative Example 5 | 0.3 | 0.5 | Continued flow |

Referring to Table 1, it can be seen that the total lengths of the coating films were in a range of 6 to 8 cm in the case of the aqueous compositions for a hard capsule prepared in Examples 1 to 5 of the present invention. In this case, since the aqueous compositions had a suitable degree of gelation to produce capsules, the aqueous compositions had improved moldability, thereby producing a high-quality hard capsule.

On the other hand, it was revealed that the total lengths of the coating films were shorter than those of Examples of the present invention in the case of the aqueous composition of Comparative Example 1 including an excessive amount of the gelation agent and the aqueous composition of Comparative Example 4 including excessive amounts of the gelation agent and gelation auxiliary agent, indicating that the aqueous compositions had a very high degree of gelation. When the aqueous composition for a hard capsule had such a high degree of gelation, the viscosity of the aqueous composition for a hard capsule was high, and thus the moldability was deteriorated. Also, the hard capsule produced using the aqueous composition for a hard capsule was easily broken due to low elongation at break and high brittleness, and thus product qualities were deteriorated. Also, in the case of the aqueous compositions of Comparative Examples 2 and 3 having a low concentration of the gelation auxiliary agent, the hard capsules were formed to have a very thin film thickness, resulting in deteriorated product qualities. Since the aqueous composition of Comparative Example 5 including no alcohol continued to flow, the aqueous composition was not produced into capsules.

### Experimental Example 2: Evaluation of solubility of hard capsule

Mold pins kept at room temperature were dipped into the aqueous composition (temperature: 60°C) prepared in Examples 1 to 5 and Comparative Examples 1 to 4. Thereafter, the mold pins (commercially available from Technophar Equipment and Service Ltd.; pin, #0) were removed from the aqueous compositions, kept under conditions of 25°C and 55% relative humidity (RH) for an hour, and then dried to remove solvent components in the aqueous compositions, thereby obtaining hard capsules. As listed in Table 1, since the composition prepared in Comparative Example 5 continued to flow, it was difficult to produce a capsule. As a result, the composition of Comparative Example 5 was excluded from the solubility evaluation.

### (Evaluation of solubility time solubility time of hard capsule)

50 ml of water (purified water) was put into an Erlenmeyer flask, and a temperature of the water was maintained at 37°C. Thereafter, each of the hard capsules was put into the Erlenmeyer flask, and a dissolved state of each of the hard capsules was checked while the Erlenmeyer flask was intermittently shaken. A time (that is, a dissolution time) elapsed from a point of time at which each of the hard capsules was put into the Erlenmeyer flask to a point of time at which each of the hard capsules was completely dissolved was recorded. The recorded elapsed times are listed in the following Table 2. Here, the hard capsule having a shorter dissolution time means that the hard capsule has a faster solubility time.

**[Table 2]**

| | Examples | | | | | Comparative Examples | | | |
|---|---|---|---|---|---|---|---|---|---|
| | 1 | 2 | 3 | 4 | 5 | 1 | 2 | 3 | 4 |
| Solubility time | 6 minutes and 20 seconds | 6 minutes and 15 seconds | 6 minutes and 5 seconds | 6 minutes and 10 seconds | 6 minutes and 30 seconds | 6 minutes and 50 seconds | 6 minutes and 20 seconds | 7 minutes and 5 seconds | 20 minutes and 26 seconds |

Referring to Table 2, it can be seen that the hard capsules produced using the aqueous compositions for a hard capsule prepared in Examples 1 to 5 had improved hard capsule solubility, compared to the hard capsules produced using the aqueous compositions of Comparative Examples 1 and 3 including an excessive amount of the gelation agent, and thus performed primary functions since the components in the capsules were dissolved within an appropriate time upon drug taking. In particular, it was revealed that the aqueous composition of Comparative Example 4 including excessive amounts of the gelation agent and the gelation auxiliary agent had a significantly long solubility time, and thus exhibited a poor solubility characteristic. Also, it was revealed that the aqueous composition of Comparative Example 2 had a short solubility time since the gelation agent and the gelation auxiliary agent were added at a small amount. However, it was revealed that the aqueous compositions of Comparative Examples had a significantly low degree of gelation, compared to the aqueous compositions of the present invention, as listed in Table 1.

While the invention has been described with reference to exemplary embodiments illustrated in accompanying drawings, these should be considered in a descriptive sense only, and it will be understood by those skilled in the art that various alterations and equivalent other embodiment may be made. Therefore, the scope of the invention is defined by the appended claims.

## Claims

1. An aqueous composition for a hard capsule comprising:
a first ingredient composed of a water-soluble cellulose ether, an alcohol, and water; and
a second ingredient comprising a gelation agent and a gelation auxiliary agent,
wherein a coating film, which is formed while the aqueous composition for a hard capsule flows down when a mold pin (60, 14.5 cm) kept at room temperature is dipped into the aqueous composition for a hard capsule to a depth of 2.5 cm, removed therefrom and flipped over, has a total length of 6.0 to 8.0 cm.

2. The aqueous composition for a hard capsule of claim 1, wherein a concentration of the gelation agent is in a range of 0.2 to 0.5 parts by weight, both exclusive, based on 100 parts by weight of the first ingredient, and a concentration of the gelation auxiliary agent is in a range of 0.3 to 0.6 parts by weight, both exclusive, based on 100 parts by weight of the first ingredient.

3. The aqueous composition for a hard capsule of claim 1, wherein the gelation agent comprises at least one water-soluble gum selected from the group consisting of carrageenan, gellan gum, xanthan gum, and pectin.

4. The aqueous composition for a hard capsule of claim 1, wherein the gelation auxiliary agent comprises at least one selected from the group consisting of potassium chloride, potassium acetate, and calcium chloride.

5. The aqueous composition for a hard capsule of claim 1, wherein a concentration of the water-soluble cellulose ether is in a range of 10 to 25% by weight, based on 100% by weight of the first ingredient.

6. The aqueous composition for a hard capsule of claim 1, wherein a concentration of the alcohol is in a range of 5 to 30% by weight, based on 100% by weight of the first ingredient.

7. The aqueous composition for a hard capsule of claim 1, wherein the water-soluble cellulose ether comprises at least one selected from the group consisting of hydroxypropyl methyl cellulose (HPMC), hydroxyethyl methyl cellulose (HEMC), and methyl cellulose (MC).

8. The aqueous composition for a hard capsule of claim 1, wherein the alcohol comprises at least one selected from the group consisting of ethanol, methanol, isopropanol, and butanol.

9. The aqueous composition for a hard capsule of claim 1, wherein the second ingredient further comprises a plasticizer at 0.05 to 5.0 parts by weight, both exclusive, based on 100 parts by weight of the first ingredient.

10. The aqueous composition for a hard capsule of claim 9, wherein the plasticizer comprises at least one selected from the group consisting of glycerol, sorbitol, propylene glycol, and polyethylene glycol.

11. A hard capsule produced using the aqueous composition for a hard capsule defined in any one of claims 1 to 10.
